# EUROPÄISCHE PATENTSCHRIFT

(11) **EP 3 194 410 B1**
(45) Veröffentlichungstag und Bekanntmachung des Hinweises auf die Patenterteilung: **13.03.2024**
(21) Anmeldenummer: 15805099.7
(22) Anmeldetag: 07.09.2015
(51) Int. Cl.: C01B 33/00, C07F 7/20, C01B 33/107, C01B 33/04, C07D 323/00

(54) **VERFAHREN ZUR AUFREINIGUNG HALOGENIERTER OLIGOSILANE**
METHOD FOR PURIFYING HALOGENATED OLIGOSILANES
PROCÉDÉ DE PURIFICATION D'OLIGOSILANES HALOGÉNÉS

(30) Priorität: 08.09.2014 DE 102014013250
(43) Veröffentlichungstag der Anmeldung: 26.07.2017
(73) Patentinhaber: Bauch, Christian, 06774 Muldenstein (DE)
(72) Erfinder: BAUCH, Christian, 06774 Muldestausee / OT Muldenstein (DE); HOLL, Sven, 65558 Gückingen (DE); HEUER, Matthias, 06792 Sandersdorf / Brehna OT Ramsin (DE)
(74) Vertreter: Dinter, Tilo
(86) Internationale Anmeldenummer: PCT/DE2015/000439
(87) Internationale Veröffentlichungsnummer: WO 2016/037601

(56) Entgegenhaltungen:
- WO-A1-2011/006695
- DE-A1- 2 852 598
- US-A- 4 985 579
- DATABASE WPI Week 198626 Thomson Scientific, London, GB; AN 1986-165094 XP002753209, -& JP S61 97129 A (MITSUI TOATSU CHEM INC) 15. Mai 1986 (1986-05-15)

## Beschreibung

Die vorliegende Erfindung betrifft ein Verfahren zur Aufreinigung halogenierter Oligosilane als reine Verbindung oder Gemisch von Verbindungen mit jeweils mindestens einer direkten Bindung Si-Si, deren Substituenten ausschließlich aus Halogen oder aus Halogen und Wasserstoff bestehen und in deren Zusammensetzung das Atomverhältnis Substituent: Silicium mindestens 3:2 beträgt.

### Stand der Technik

DE 28 52 598 A1 offenbart ein Verfahren zur Reinigung von Chlorsilanen zur Entfernung von Phosphor-, Arsen-, Antimonverunreinigungen durch Adsorption an Aluminium-trifluorid.

WO 2011/006695 A1 offenbart ein Verfahren zur Behandlung einer Zusammensetzung, enthaltend mindestens ein Silan und mindestens ein Fremdmetall und/oder eine Fremdmetall enthaltende Verbindung, dadurch gekennzeichnet, dass die Zusammensetzung mit mindestens einem organischen, aminofunktionalisierten, polymeren Adsorptionsmittel in Kontakt gebracht wird und Gewinnen der Zusammensetzung, in der der Gehalt an Fremdmetall und/oder der Fremdmetall enthaltenden Verbindung vermindert ist.

US 4 985 579 A offenbart ein Verfahren zur Aufreinigung von Organosilanen durch den Kontakt mit Halogenwasserstoff, bevorzugt HCl.

Aus ADTABASE WPIWeek 198626 Thomson Scientific, London, GB;AN 1986-165094-& JP S61 97129 A (MITSUI TOATSU CHEM INC) 15. Mai 1986 (1986-05-15) ist ein Verfahren zur Aufreinigung von Fluorsilanen bekannt, bei dem Verunreinigungen (Chlor - Silane) durch die Behandlung mit Metallfluorid/Aktivkohle vermindert werden.

Andere Verfahren zur Entfernung spezieller Kontaminationen aus halogenierten Silanen sind aus dem Stand der Technik bekannt:
In DE1020090277291 wird ein Verfahren offenbart, dass Kontaminationen in Silanen durch Verbindungen metallischer oder metalloider Elemente reduziert, indem den betreffenden Silanen organische Verbindungen als Fängerreagenzien zugesetzt werden, welche Aminogruppen enthalten. Diese Aminogruppen fungieren als Liganden und koordinieren sich an die Metall- oder Metalloidzentren. Neben Metallenverbindungen wie Eisenchloriden können auf diese Weise auch Borverbindungen entfernt werden.

Van Dyke et al. (Inorg.Chem., Vol. 3, No.5, 1964, S. 747 - 752) beschreibt die Reaktion von Bortrichlorid mit verschiedenen Silylsiloxanen, wobei diese zu Silylchloriden gespalten werden.

### Nachteile des Standes der Technik

Das in DE102009027729A1 vorgestellte Verfahren ist nicht zur Aufreinigung halogenierter Oligosilane geeignet, da dieses Verfahren die Zugabe organischer Verbindungen mit Aminogruppen zu den Produktgemischen voraussetzt. Solche Reagenzien können im Falle von Oligosilanen nicht eingesetzt werden, da bekannt ist, dass Aminogruppen Skelettumlagerungen bei halogenierten Oligosilanen bewirken, wobei einerseits kürzerkettige Halogensilane, z.B. SiCl4, andererseits längerkettige stark verzweigte Halogensilane, z.B. (SiCl₃)₄Si, entstehen. Dies kann die vollständige Zersetzung des gewünschten Produktes zur Folge haben, weshalb diese Methode für halogenierte Oligosilane nicht genutzt werden kann.

Die Methode von Van Dyke et al. betrifft lediglich die Entfernung verschiedener Siloxane, nicht jedoch die Reduzierung einer Kontamination durch Verbindungen metallischer oder metalloider Elemente. Überdies ist hierzu die Zugabe von BCl3 zur Produktmischung nötig, was sich für viele Anwendungen der halogenierten Oligosilane ausschließt, da Bor z.B. in der Halbleiterherstellung ein kritisches Element ist, da es als Dotierstoff wirkt.

**Tabelle 1: Abkürzungen und Synonyme**

| Abkürzungen | |
|---|---|
| Edukte | Startverbindungen für eine chemische Reaktion |
| Prekursoren | Ausgangsstoff für den jeweils betrachteten Prozess |
| [18]Krone-6 | Zyklischer Polyether mit 18 Ringatomen (davon 6 Sauerstoff) |
| OCS | chlorierte Oligosilane |
| Siloxanyl | O - SiR₃(R=Halogen und/oder H und/oder Organyl/Siloxanyl |
| Si₂Cl₆ | Hexachlordisilan (HCDS) |
| Si₂Cl₆O | Hexachlordisiloxan (HCDSO) |
| ppm≡ppmw | Gewichtsteile pro Million (parts per million by weight (10⁻⁶)) |
| ppb≡ppbw | Gewichtsteile pro Milliarde (parts per billion by weight (10⁻⁹)) |
| HCl | Salzsäuregas |
| hPa | Hektopascal≡1mbar |

### Aufgabenstellung

Ziel ist es, ein Verfahren zur Aufreinigung halogenierter Oligosilane zur Verfügung zu stellen, welches bei geringem Material- und Kostenaufwand Spurenkontaminationen, insbesondere metallhaltige Verbindungen, sicher und schnell aus dem Produkt entfernt, ohne die Produktausbeuten an OCS signifikant zu reduzieren. Hierbei soll die Reinheit besonders im Hinblick auf Kontaminationen durch Dotierstoffe und Metallverbindungen und andere für die Anwendung im Halbleiterbereich schädliche Elemente gesteigert werden.

### Lösung der Probleme des Standes der Technik

Diese Aufgabe wird durch die in Anspruch 1 aufgeführten Merkmale Verfahren zur Erhöhung der Reinheit halogenierter Oligosilane SiₙX₂ₙ₊₂ mit n=2 bis n=6 als reine Verbindung oder Gemisch von Verbindungen, deren Substituenten X Chlor oder Chlor und Wasserstoff umfassen, **durch Reduzierung von Metallen und Dotierstoffen**, dadurch gekennzeichnet, dass
1. dem halogenierten Oligosilan Kronenether und ein Fluorid oder ein Fluoridgemisch zugesetzt werden, wobei von der konkreten Verbindung Fluorid F⁻ für eine Reaktion zur Verfügung gestellt wird, in einer Menge, gerechnet als F⁻ in Bezug auf die Masse des halogenierten Oligosilans, von mehr als 1ppb,
2. mindestens eine Prozessierungsphase folgt, in der mindestens das Fluorid auf das halogenierte Oligosilan einwirkt, wobei die mindestens eine Prozessierung ausgewählt ist aus einer Gruppe, welche Rühren, Schwenken, Schütteln, unter Rückfluss erhitzen, Diffundieren und Hindurchleiten
   umfasst, ,
3. mindestens ein Trennverfahren zur Isolierung des halogenierten Oligosilans folgt, ausgewählt aus einer Gruppe, welche Dekantieren, Filtrieren, Destillieren und Sublimieren umfasst gelöst.
   Das halogenierte Oligosilan weist nach der Aufreinigung einen reduzierten Gesamtmetallgehalt auf.

Die mit dem erfindungsgemäßen Verfahren erzielten Vorteile zur Aufreinigung chlorierter Oligosilane bestehen insbesondere darin, dass durch die Verwendung eines Aufreinigungsmittels Kontaminationen gezielt gebunden werden, während im Stand der Technik sehr langwierige und teure Destillationsschritte nötig sind, um die für die Halbleitertechnik nötige Reinheit zu erreichen.

Eine vorteilhafte Ausgestaltung **ist wie folgt** angegeben: Verfahren nach Anspruch 1, dadurch gekennzeichnet, dass das Fluorid ausgewählt wird aus einer Gruppe, welche Alkalimetallfluoride, Erdalkalimetallfluoride, Siliciumfluoride SiₙXₒFₚ (X=Halogen, Organyl, Siloxanyl und/oder Wasserstoff; o+p=2n+2; n≥1) und Zinkfluorid umfasst, bevorzugt Alkalimetallfluoride, besonders bevorzugt Kaliumfluorid.

Die verwendeten Fluoride sollten kostengünstig sein und sich im geplanten Trennverfahren leicht wieder entfernen lassen. Weiterhin sollten die jeweiligen zusätzlichen Komponenten wie Gegenionen keine unerwünschten Reaktionen mit dem OCS eingehen, weshalb sich z.B. Kaliumfluorid anbietet.

Der Fachmann erkennt leicht, dass bei der Wahl der Fluoride aus vielen verschiedenen Verbindungen ausgewählt werden kann, wobei es jedoch im Grunde nur darauf ankommt, dass von der konkreten Verbindung Fluorid (F⁻) für die Reaktion zur Verfügung gestellt werden kann. Daher können auch andere Fluoride die hier erwähnten ganz oder teilweise ersetzen, ohne dass das Wesen des erfindungsgemäßen Verfahrens verlassen wird.

Eine weitere vorteilhafte Ausgestaltung ist nachfolgend angegeben:
Verfahren nach Anspruch 1, dadurch gekennzeichnet, dass **der erfindungsgemäß** dem halogenierten Oligosilan **zugegebene** Komplexbildner **der Kronenether** [18]Krone-6 ist.

Komplexbildner können eine große Anzahl von Metallionen binden und auf diese Weise dem Austausch mit dem gewünschten Produkt entziehen. Hierzu sollte die Komplexbildungskonstante möglichst groß und die konkrete Verbindung nicht zu teuer sein. Auch hier sollten sich die verwendeten Komplexbildner im geplanten Trennverfahren leicht wieder entfernen lassen und keine unerwünschten Reaktionen mit dem OCS eingehen. Beides trifft beispielsweise auf Kronenether wie [18]Krone-6 zu.

Eine weitere vorteilhafte Ausgestaltung ist nachfolgend angegeben:
Verfahren nach Anspruch 1, dadurch gekennzeichnet, dass dem halogenierten Oligosilan und/oder dem Fluorid vor oder während der Prozessierungsphase ein Lösungsmittel zugesetzt wird, ausgewählt aus einer Gruppe, welche Alkane, Cycloalkane, Ether, Aromaten und chlorierte Silane umfassst, bevorzugt Cycloalkane und Ether wie Triglyme, Diglyme, Dioxan, Dibutylether, THF und Diethylether, besonders bevorzugt Cycloalkane, insbesondere Cyclohexan, wobei der Anteil des Lösungsmittels im Gemisch mit den halogenierten Oligosilanen mindestens 0,01 Massen%, bevorzugt mindestens 0,1 Massen%, besonders
bevorzugt mindestens 1 Massen%, insbesondere mindestens 2 Massen% ist.

Die Zugabe von Lösungsmitteln kann vorteilhaft sein, um z.B. eine löslichkeitsvermittelnde Wirkung zu entfalten. Dies besonders im Hinblick auf die aufreinigenden Additive, welche im erfindungsgemäßen Verfahren eingesetzt werden. Die verwendeten Lösungsmittel müssen hierzu möglichst inert und leicht von den Produkten abzutrennen sein.

Eine weitere vorteilhafte Ausgestaltung ist nachfolgend angegeben:
Verfahren nach Anspruch 1 dadurch gekennzeichnet, dass X in SiₙX₂ₙ₊₂ zu mehr
als 95 Atom% Chlor ist, bevorzugt zu mehr als 98 Atom%,
   und/oder der
Wasserstoffgehalt in SiₙX₂ₙ₊₂ kleiner als 5 Atom%, bevorzugt kleiner als 2
Atom%, besonders bevorzugt kleiner als 1 Atom% ist.

Die wichtigsten Zielverbindungen des erfindungsgemäßen Verfahrens sind hochchlorierte Oligosilane, wobei aber auch anders halogenierte oder teilhydrierte OCS umgesetzt werden, wenn diese flüssig/verflüssigt sind, oder in Lösung vorliegen.

Eine weitere vorteilhafte Ausgestaltung ist nachfolgend angegeben:
Verfahren nach dem Anspruch 1 dadurch gekennzeichnet, dass das chlorierte Oligosilan ein Verdünnungsmittel enthält, ausgewählt aus einer Gruppe umfassend SiCl₄, Si₂Cl₆, Si₃Cl_{8,} Si₄Cl₁₀, wobei der Anteil des Verdünnungsmittels am
halogenierten Oligosilan mindestens 0,001 Massen%, bevorzugt mindestens 0,1 Massen%, besonders bevorzugt mindestens 1Massen%, insbesondere mindestens 10 Massen% ist.

Die Zugabe von Verdünnungsmitteln kann dann ratsam sein, wenn das OCS fest oder zu viskos ist, um eine effiziente Prozessierung zu erlauben.

Verdünnngsmittel können im Unterschied zu den oben erwähnten Lösungsmitteln auch in großer Menge eingesetzt werden, wenn es sich wie beschrieben um Verbindungen handelt, die mit den erfindungsgemäßen OCS chemisch verwandt sind und daher keine störenden Kontaminationen in das Produkt einbringen.

Eine weitere vorteilhafte Ausgestaltung ist nachfolgend angegeben:
Verfahren nach Anspruch ,1 dadurch gekennzeichnet, dass mindestens ein Trennverfahren bei einem Druck kleiner als 1600hPa, bevorzugt kleiner als 800hPa, besonders bevorzugt kleiner als 80hPa, insbesondere kleiner als 1hPa betrieben wird.

Trennverfahren wie z.B. die Destillation sollten vorteilhafterweise bei reduzierten Drücken durchgeführt werden, um Zersetzungserscheinungen in Gegenwart des Aufreinigungsmittels zu vermeiden. Die Drücke müssen dabei umso niedriger sein, je höher das betreffende OCS siedet, um eine hinreichende Reduzierung des Siedepunktes zu erzielen.

Eine weitere vorteilhafte Ausgestaltung ist nachfolgend angegeben:
Verfahren nach Anspruch 1 dadurch gekennzeichnet, dass mindestens ein Prozessierungsschritt und/oder ein Trennverfahren bei einer Temperatur von mehr als 30°C, bevorzugt sogar mehr als 50°C, besonders bevorzugt mehr als 80°C, insbesondere mehr als 100°C, betrieben wird.

Eine Erhöhung der Temperatur ist vorteilhaft, um eine schnellere Reaktion mit den Aufreinigungsstoffen zu erreichen. Weiterhin ist es bei längerkettigcn OCS sinnvoll, in der Wärme zu arbeiten, um deren Viskosität zu reduzieren.

Eine weitere vorteilhafte Ausgestaltung ist nachfolgend angegeben:
Verfahren nach Anspruch 1 dadurch gekennzeichnet, dass dem halogenierten Oligosilan vor oder während der Prozessierungsphase ein Siloxan als reine Verbindung oder Gemisch von Verbindungen, ausgewählt aus der Gruppe Cl₃SiOSiCl₃, Cl₃SiOSiCl₂SiCl₃, Cl₃SiOSiCl₂OSiCl₃, zugesetzt wird, wobei der Anteil des Siloxans im Gemisch mit dem halogenierten Oligosilan mindestens 0,001 Massen%, bevorzugt mindestens 0,01 Massen%, besonders bevorzugt mindestens 0,1 Massen%, insbesondere mindestens 1 Massen% beträgt.

Das erfindungsgemäße Verfahren sieht hier eine weitere Verfahrensvariante vor, bei der den Edukten halogenierte Disiloxane zugesetzt werden, um eine Aufreinigungswirkung im Verlaufe des Gesamtprozesses mit Hinblick auf Metallverbindungen und Dotierstoffe wie Bor und Phosphor zu erreichen. Die Disiloxane binden hierbei während der verschiedenen Verfahrensschritte nach Anspruch l einen erheblichen Teil der Metall- und Dotierstoffkontaminationen in Form von Oxiden oder gemischten Oxiden, welche z.B. Si-O Gruppen enthalten, wodurch diese bei den erfindungsgemäßen Trennverfahren, wie z.B. einer Destillation als schwerflüchtige Rückstände abgetrennt werden können. Zweckmäßigerweise werden die Disiloxane zu Anfang des Verfahrens zugemischt, wobei diese z.B. in SiC14 gelöst oder flüssig vorgelegt werden können. Dadurch kann sich der aufreinigcnde Effekt, den diese Komponenten bewirken bereits vom ersten Prozessienmgsschritt an entfalten, wie dies erfindungegemäß vorgesehen ist. Speziell die Disiloxane können aber auch zu einem späteren Zeitpunkt dem erfindungsgemäßen Verfahren beigemischt werden, wobei eine gewisse aufreinigende Wirkung weiterhin entfaltet wird. Wie der Fachmann leicht erkennt, wird hierdurch das offenbarte Prinzip des erfindungsgemäßen Verfahrens nicht verlassen.

Die Menge des Aufreinigungsadditivs hängt von der Art und Menge der zu entfernenden Kontaminationen ab. Tendenziell führt eine größere Menge an Disiloxanen zu einer besseren Aufreinigung. Jedoch werden die Disiloxane ebenfalls abhängig von deren Dampfdruck im Prozess mitgeschleppt und können im Endprodukt stören, weshalb sie bevorzugt im letzten Schritt, z.B. durch Destillation weitgehend entfernt werden müssen. Dies gelingt umso besser, je weniger von vornherein vorhanden war, so dass man hier in der Regel einen Kompromiss zwischen Reinigungswirkung und erträglichem Restgehalt im Endprodukt wählen wird. Es ist vorteilhaft, abhängig vom Siedepunkt des gewünschten Endproduktes ein Disiloxan zu wählen, welches einen hierzu möglichst unterschiedlichen Siedepunkt besitzt, um den abschließenden Aufreinigungsschritt nicht zu erschweren.

### Gewerbliche Anwendbarkeit

Das erfindungsgemäße Verfahren zur Aufreinigung halogenierter Oligosilane bietet einen signifikanten ökonomischen Vorteil, da sowohl die Ausbeute an Produkten als auch die pro-Zeit-Ausbeute durch Zugabe der erfindungsgemäßen Additive gegenüber dem Stand der Technik erheblich gesteigert werden können.

Weiterhin wird die Reinheit der Endprodukte durch Zusatz der erfindungsgemäßen Aufreinigungsmittel erhöht, was zu einer verbesserten Position des Produktes am Markt führt, wodurch ein weiterer ökonomischer Vorteil innerhalb eines Verfahrens zur Aufreinigung chlorierter Oligosilane (OCS) erreicht wird.

Für die technische Realisierung des Verfahrens kommen verschiedene Lösungen in Frage, aus denen in den unten aufgeführten Ausführungsbeispielen nicht-ausschließliche Varianten für das vorliegende Problem aufgeführt sind.

### Ausführungsbeispiel 1

70kg Hexachlordisilan werden mit einer Suspension von 18g [18]Krone-6 und 2,7g KF in 50mL Cyclohexan versetzt. Die Mischung wird 2h gerührt und anschließend fraktionierend destilliert. Nach zehn Stunden werden im Hauptlauf 62kg HCDS erhalten. Dabei wurden im Verlauf dieser Aufreinigung der ursprüngliche Aluminiumgehalt von 3,8ppm und der Eisengehalt von 1,3ppm jeweils auf unter 30ppb reduziert.

### Ausführungsbeispiel 2

60kg Hexachlordisilan werden mit einer Suspension von 50g [18]Krone-6 und 5g KF in 150mL Triglyme versetzt. Die Mischung wird über Nacht gerührt und anschließend fraktionierend destilliert. Nach neun Stunden werden im Hauptlauf 45kg HCDS erhalten. Dabei wurden im Verlauf dieser Aufreinigung der ursprüngliche Aluminiumgehalt von 800ppb, der Eisengehalt von 75ppb und der Magnesiumgehalt von 180ppb jeweils auf unter 5ppb reduziert.

### Ausführungsbeispiel 3

8kg Oktachlortrisilan werden mit einer Suspension von 0,5g NaF und 4,5g [15]Krone-5 in 30mL Dichlormethan versetzt. Die Mischung wird über Nacht bei RT gerührt und anschließend von ungelöstem Feststoff dekantiert. Das OCTS wird nun unter Vakuum (ca. 10hPa) zügig über eine kurze Kolonne abdestilliert und anschließend unter Vakuum (ca. 10hPa) fraktionierend destilliert. Innerhalb von 8 Stunden werden im Hauptlauf 5kg OCTS erhalten. Die Gehalte beispielhafter Spurenverunreinigungen in ppb sind in nachfolgender Tabelle jeweils vor und nach Aufreinigung dargestellt.

| OCTS | Al | Cr | Mn | Cu | Fe | Mg | K | Na | Ti | Ca |
|---|---|---|---|---|---|---|---|---|---|---|
| roh | 920 | <30 | <30 | 58 | 46 | 110 | 160 | 120 | <30 | 750 |
| gereinigt | <30 | <30 | <30 | <30 | <30 | <30 | <50 | <50 | <30 | <50 |

### Ausführungsbeispiel 4

1,5kg eines Gemisches aus ca. 90% Tetrachlordisilan-Isomerengemisch und 10% Pentachlordisilan werden mit einer Suspension von 0,1g NaF und 1g [12]Krone-4 in 20mL Dichlormethan versetzt. Die Mischung wird 2h bei RT und anschließend über Nacht bei 0°C gerührt. Die flüssige Phase wird von ungelöstem Feststoff dekantiert, durch eine Glasfilterfritte (D3) filtriert und anschließend unter Vakuum (ca. 10hPa) zügig über eine kurze Kolonne umkondensiert. Das Kondensat wird anschließend unter Vakuum (ca. 10hPa) fraktionierend destilliert. Innerhalb von 14 Stunden werden im Hauptlauf 1,2kg Oligosilangemisch erhalten. Die Gehalte beispielhafter Spurenverunreinigungen in ppb sind in nachfolgender Tabelle nach der Aufreinigung dargestellt.

| TCDS/PCDS | Al | Cr | Mn | Cu | Fe | Mg | K | Na | Ti | Ca |
|---|---|---|---|---|---|---|---|---|---|---|
| gereinigt | <30 | <30 | <30 | <30 | <30 | <30 | <50 | <50 | <30 | <50 |

### Ausführungsbeispiel 5

35kg Hexachlordisilan werden mit einer Suspension von 3g KF, 12g [15]Krone-5 und 15g [18]Krone-6 in 120mL Dichlormethan versetzt. Die Mischung wird innerhalb von ca. 2h bis zum Reflux erhitzt und anschließend fraktionierend destilliert. Innerhalb von 8 Stunden werden im Hauptlauf 28kg HCDS erhalten. Die Gehalte beispielhafter Spurenverunreinigungen in ppb sind in nachfolgender Tabelle jeweils vor und nach Aufreinigung dargestellt.

| HCDS | Al | B | Sb | Cu | Fe | Mg | K | Na | Ti | Ca |
|---|---|---|---|---|---|---|---|---|---|---|
| roh | 112 | 420 | 0.7 | 5.2 | 848 | 11 | 78 | 49 | 734 | 125 |
| gereinigt | 25 | 23 | 0.1 | 0.8 | 13 | 7 | 6.6 | 2.2 | 27 | 26 |

### Ausführungsbeispiel 6

40kg Hexachlordisilan werden mit einer Suspension von 3,5g KF, 14g [15]Krone-5 und 17g [18]Krone-6 in 140mL Dichlormethan versetzt. Die Mischung wird innerhalb von ca. 2h bis zum Reflux erhitzt und anschließend fraktionierend destilliert. Innerhalb von 10 Stunden werden im Hauptlauf 32kg HCDS erhalten. Hiervon werden 26kg HCDS mit einer Suspension aus 2,2g KF, 11g [18]Krone-6 und 10g [12]Krone-4 in 100mL Dichlormethan versetzt und erneut fraktionierend destilliert. Nach 9 Stunden werden im Hauptlauf 20kg HCDS isoliert. Die Gehalte beispielhafter Spurenverunreinigungen in ppb sind in nachfolgender Tabelle jeweils vor und nach Aufreinigung dargestellt.

| HCDS | Al | B | Sb | Cu | Fe | Mg | K | Na | Ti | Ca |
|---|---|---|---|---|---|---|---|---|---|---|
| roh | 55 | 57 | 0.9 | 4.5 | 21 | 8.1 | 39 | 133 | 342 | 77 |
| gereinigt | 3.5 | 2.4 | 0.1 | 0.1 | 1.6 | 0.8 | 0.07 | 0.9 | 2.8 | 6.5 |

## Patentansprüche

1. Verfahren zur Erhöhung der Reinheit halogenierter Oligosilane SiₙX₂ₙ₊₂ mit n=2 bis n=6 als reine Verbindung oder Gemisch von Verbindungen, deren Substituenten X Chlor oder Chlor und Wasserstoff umfassen, **durch Reduzierung von Metallen und Dotierstoffen, dadurch gekennzeichnet, dass**
(a) dem halogenierten Oligosilan **Kronenether und** ein Fluorid oder **ein Fluoridgemisch** zugesetzt werden, wobei von der **konkreten Verbindung Fluorid** F⁻ **für eine Reaktion zur Verfügung gestellt wird**, in einer Menge, gerechnet als F⁻ in Bezug auf die Masse des halogenierten Oligosilans, von mehr als 1 ppb zugegeben wird,
(b) mindestens eine Prozessierungsphase folgt, in der mindestens das Fluorid auf das halogenierte Oligosilan einwirkt, wobei die mindestens eine Prozessierung ausgewählt ist aus einer Gruppe, welche Rühren, Schwenken, Schütteln, unter Rückfluss erhitzen, Diffundieren und Hindurchleiten umfasst,
(c) mindestens ein Trennverfahren zur Isolierung des halogenierten Oligosilans folgt, ausgewählt aus einer Gruppe, welche Dekantieren, Filtrieren, Destillieren und Sublimieren umfasst.

2. Verfahren nach Anspruch 1, **dadurch gekennzeichnet, dass** das Fluorid ausgewählt wird aus einer Gruppe, welche Alkalimetallfluoride, Erdalkalimetallfluoride, Siliciumfluoride SiₙXₒFₚ (X=Halogen, Organyl, Siloxanyl und/oder Wasserstoff; o+p=2n+2; n≥1) und Zinkfluorid umfasst.

3. Verfahren nach Anspruch 1, **dadurch gekennzeichnet, dass** dem halogenierten Oligosilan und/oder dem Fluorid vor oder während der Prozessierungsphase ein Lösungsmittel zugesetzt wird, ausgewählt aus einer Gruppe, welche Alkane, Cycloalkane, Ether, Aromaten und chlorierte Silane umfasst, wobei der Anteil des Lösungsmittels im Gemisch mit den halogenierten Oligosilanen mindstens 0,01 Massen% ist.

4. Verfahren nach Anspruch 1 **dadurch gekennzeichnet, dass** X in SiₙX₂ₙ₊₂ zu mehr als 95 Atom% Chlor ist und/oder
der Wasserstoffgehalt in SiₙX₂ₙ₊₂ kleiner als 5 Atom% ist.

5. Verfahren nach Anspruch 1 **dadurch gekennzeichnet, dass** das chlorierte Oligosilan ein Verdünnungsmittel enthält, ausgewählt aus einer Gruppe umfassend SiCl₄, Si₂Cl₆, Si₃Cl₈, Si₄Cl₁₀, wobei der Anteil des Verdünnungsmittels am halogenierten Oligosilan mindestens 0,001 Massen% ist.

6. Verfahren nach Anspruch 1 **dadurch gekennzeichnet, dass** mindestens ein Trennverfahren bei einem Druck kleiner als 1600hPa betrieben wird.

7. Verfahren nach Anspruch 1 **dadurch gekennzeichnet, dass** mindestens ein Prozessierungsschritt und/oder ein Trennverfahren bei einer Temperatur von mehr als 30°C betrieben wird.

8. Verfahren nach Anspruch 1 **dadurch gekennzeichnet, dass** dem halogenierten Oligosilan vor oder während der Prozessierungsphase ein Siloxan als reine Verbindung oder Gemisch von Verbindungen, ausgewählt aus der Gruppe Cl₃SiOSiCl₃, Cl₃SiOSiCl₂SiCl₃, Cl₃SiOSiCl₂OSiCl₃, zugesetzt wird, wobei der Anteil des Siloxans im Gemisch mit dem halogenierten Oligosilan mindestens 0,001 Massen% beträgt.

## Claims

1. Process for increasing the purity of halogenated oligosilanes SiₙX₂ₙ₊₂ with n=2 to n=6 as a pure compound or mixture of compounds whose substituents X comprise chlorine or chlorine and hydrogen, by reducing metals and dopants, **characterized in that**
(a) a crown ether and a fluoride or a fluoride mixture is added to the halogenated oligosilane, wherein fluoride F⁻ from the specific compound is made available for a reaction, being admixed in an amount of more than 1 ppb calculated as F⁻ in relation to the mass of the halogenated oligosilane,
(b) at least one processing phase follows, in which at least the fluoride reacts on the halogenated oligosilane, wherein the at least one processing is selected from a group comprising stirring, swirling, shaking, heating under reflux, diffusing, and passing through,
(c) at least one separation method for the isolation of the halogenated oligosilane follows, selected from a group comprising decanting, filtering, distilling, and subliming.

2. Method according to claim 1, **characterized in that** the fluoride is selected from a group comprising alkali metal fluorides, alkaline earth metal fluorides, silicon fluorides SiₙX₀Fₚ (X = halogen, organyl, siloxanyl and/or hydrogen; o+p=2n+2; n≥1), and zinc fluoride.

3. Method according to claim 1, **characterized in that** to the halogenated oligosilane and/or to the fluoride is added before or during the processing phase a solvent that is selected from a group comprising alkanes, cycloalkanes, ethers, aromatics, and chlorinated silanes, wherein the proportion of the solvent in the mixture with the halogenated oligosilanes is at least 0.01 mass%.

4. Method according to claim 1, **characterized in that** X in SiₙX₂ₙ₊₂ is more than 95 atom% chlorine and/or the hydrogen content in SiₙX₂ₙ₊₂ is less than 5 atom%.

5. Method according to claim 1, **characterized in that** the chlorinated oligosilane contains a diluent selected from a group comprising SiCl₄, Si₂Cl₆, Si₃Cl₈, Si₄Cl₁₀, wherein the proportion of the diluent based on the halogenated oligosilane is at least 0.001 mass%.

6. Method according to claim 1, **characterized in that** at least one separation process is operated at a pressure less than 1600 hPa.

7. Method according to claim 1, **characterized in that** the at least one processing step and/or one separation process is operated at a temperature of more than 30 °C.

8. Method according to claim 1, **characterized in that** a siloxane as a pure compound or mixture of compounds, selected from the group Cl₃SiOSiCl₃, Cl₃SiOSiCl₂SiCl₃, Cl₃SiOSiCl₂OSiCl₃, is added to the halogenated oligosilane before or during the processing phase, wherein the proportion of the siloxane in the mixture with the halogenated oligosilane is at least 0.001 mass%.

## Revendications

1. Procédé permettant d'augmenter la pureté d'oligosilanes halogénés SiₙX₂ₙ₊₂ avec n = 2 à n = 6 en tant que composé pur ou mélange de composés, dont les substituants X comprennent le chlore ou le chlore et l'hydrogène, par réduction de métaux et de dopants, **caractérisé en ce que**
(a) un éther couronne et un fluorure ou un mélange de fluorures sont ajoutés à l'oligosilane halogéné, dans lequel le fluorure F⁻ est fourni par le composé concret pour une réaction et est ajouté en une quantité, calculée en tant que F⁻, par rapport à la masse de l'oligosilane halogéné, de plus de 1 ppb,
(b) au moins une phase de traitement suit, dans laquelle au moins le fluorure agit sur l'oligosilane halogéné, dans lequel l'au moins un traitement est choisi dans un groupe comprenant mélange, brassage, agitation, chauffage à reflux, diffusion et conduction,
(c) au moins un procédé de séparation permettant d'isoler l'oligosilane halogéné suit, choisi dans un groupe comprenant décantation, filtration, distillation et sublimation.

2. Procédé selon la revendication 1, **caractérisé en ce que** le fluorure est choisi dans un groupe comprenant fluorures de métaux alcalins, fluorures de métaux alcalino-terreux, fluorures de silicium SiₙXₒFₚ (X = halogène, organyle, siloxanyle et/ou hydrogène ; o + p = 2n + 2 ; n ≥ 1) et fluorure de zinc.

3. Procédé selon la revendication 1, **caractérisé en ce que**, avant ou pendant la phase de traitement, un solvant est ajouté à l'oligosilane halogéné et/ou au fluorure, lequel solvant est choisi dans un groupe comprenant alcanes, cycloalcanes, éthers, composés aromatiques et silanes chlorés, dans lequel la proportion de solvant dans le mélange avec les oligosilanes halogénés est d'au moins 0,01 % massique.

4. Procédé selon la revendication 1, **caractérisé en ce que** X dans SiₙX₂ₙ₊₂ est du chlore à plus de 95 % atomique et/ou
la teneur en hydrogène dans SiₙX₂ₙ₊₂ est inférieure à 5 % atomique.

5. Procédé selon la revendication 1, **caractérisé en ce que** l'oligosilane chloré contient un diluant choisi dans un groupe comprenant SiCl₄, Si₂Cl₆, SisCla, Si₄Cl₁₀, dans lequel la proportion de diluant dans l'oligosilane halogéné est d'au moins 0,001 % massique.

6. Procédé selon la revendication 1, **caractérisé en ce qu'**au moins un procédé de séparation est mis en oeuvre à une pression inférieure à 1 600 hPa.

7. Procédé selon la revendication 1, **caractérisé en ce qu'**au moins une étape de traitement et/ou un procédé de séparation sont mis en oeuvre à une température supérieure à 30 °C.

8. Procédé selon la revendication 1, **caractérisé en ce que**, avant ou pendant la phase de traitement, un siloxane est ajouté en tant que composé pur ou mélange de composés à l'oligosilane halogéné, lequel siloxane est choisi dans le groupe comprenant Cl₃SiOSiCl₃, Cl₃SiOSiCl₂SiCl₃, Cl₃SiOSiCl₂OSiCl₃, dans lequel la proportion de siloxane dans le mélange avec l'oligosilane halogéné est d'au moins 0,001 % massique.
